# EUROPÄISCHE PATENTANMELDUNG

(11) **EP 2 829 278 A1**
(43) Veröffentlichungstag der Anmeldung: **28.01.2015**
(21) Anmeldenummer: 13177718.7
(22) Anmeldetag: 23.07.2013
(51) Int. Cl.: A61K 38/17, A61P 19/04

(54) **Serum zur Behnandlung von Krankheiten**

(71) Anmelder: Scientific BioTech GmbH, 50678 Köln (DE)
(72) Erfinder: Bora, Jurate, 53604 Bad Honnef (DE); Bora, Markus, 53604 Bad Honnef (DE)
(74) Vertreter: Simandi, Claus

(57) **Zusammenfassung**

Die vorliegende Erfindung betrifft ein Verfahren zur in-vitro-Bildung samt Anreicherung von Interleukin-1-Rezeptor-Antagonisten (kurz: IRAP, IL-1-Ra) zur Behandlung von Krankheiten und dessen Verwendung als Kosmetikum.

## Beschreibung

Die vorliegende Erfindung betrifft ein Verfahren zur in-vitro-Bildung samt Anreicherung von Interleukin-1-Rezeptor-Antagonisten (kurz: IL-1-Ra) zur Behandlung von Krankheiten und dessen Verwendung als Kosmetikum.

Therapeutisch wirksame Proteine und Seren sind seit langem bekannt und als Arzneimittel beschrieben bzw. zugelassen. Von besonderer Bedeutung sind in diesem Zusammenhang autologe, d. h. körpereigene Proteine und Blutbestandteile.

Hervorzuheben sind zu den autologen Substanzen, insbesondere zu Interleukin-1-Rezeptor-Antagonisten, die beteiligten Substanzen, wie Interleukin-4, Interleukin-10, Interleukin-11 und Interleukin-13, die Gegenstand von autologen Cytokinen (anti-inflammatorische Cytokine) sind. Weiterhin sind in diesem Zusammenhang die Wachstumsfaktoren, wie PDGF, FGF, TGF-beta und VEGF beschrieben.

Die deutsche Patentanmeldung DE 42 444 37 A1 offenbart ein Verfahren zur Gewinnung körpereigener Cytokine, wobei jedoch ein Ozonsauerstoffgemisch zur Begasung des Eigenblutes verwendet wird und mittels Zentrifugation einzelne Fraktionen gewonnen werden. Auf diese Weise werden Radikale für empfindliche Zellen eliminiert. Weiterhin beschreibt das deutsche Patent DE 199 03 876 B4 ein Verfahren zur in-vitro-Bildung und Anreicherung von Interleukin-1-Rezeptor-Antagonisten, wobei eine mit Blut gefüllte Spritze bis zu 72 Stunden bei Raumtemperatur inkubiert und einem Patienten verabreicht wird. Die besagte Schrift geht ausführlich auf die Verwendung von Glasspritzen zur Verabreichung des hergestellten Serums ein. Im Stand der Technik ist bekannt, dass Interleukin-1-Rezeptor-Antagonisten zur Behandlung von Arthrose / Arthritis eingesetzt werden können, wobei die besagten Interleukin-1-Rezeptor-Antagonisten eine autoentzündliche Reaktion verhindern. Weiterhin beschreibt EP 2 123 289 A die Applikation von autologen Cytokinen bei der Behandlung von Entzündungserkrankungen an Gelenken, Muskeln, Sehnen, Bändern und perineuralen Bereichen [0014].

Überraschenderweise konnte nunmehr festgestellt werden, dass angereicherte Interleukin-1-Rezeptor-Antagonisten für weitere Indikationen einen palliativen und curativen Effekt aufweisen.

Daher betrifft die Erfindung ein Serum enthaltend Interleukin-1-Rezeptor-Antagonisten hergestellt und angereichert durch ein Verfahren mit den folgenden Schritten:
a.) Blutentnahme, vorzugsweise 5 bis 100 ml Blut mittels Butterfly oder Kanüle, wie z.B. eine Spritze;
b.) Inkubation in Gegenwart einer Oberfläche, vorzugsweise bei 36 - 38 Grad Celsius;
c.) Zentrifugation, vorzugsweise 5 Minuten bei 4.000 rpm und Abtrennen des Überstandes unter Erhalt des Serums (nachstehend: "erfindungsgemäßes Serum"), und
d.) Applikation des Serums, vorzugsweise eine lokale (topische) Applikation am Mensch oder Tier, besonders bevorzugt mittels subkutaner Applikation.

Die Blutentnahme erfolgt vorzugsweise mit einer handelsüblichen Spritze. Das entnommene Blut wird vorzugsweise in einen Container (z.B. Pyrex-Rohr) mit einer großen Oberfläche überführt. Beispielsweise kann der Container zur Vergrößerung der Oberfläche Kugeln oder Perlen enthalten. Weiterhin sind zur Erlangung von Scherkräften unebene oder raue Oberflächen bevorzugt (z.B. Kugeln, Deformationen, etc.). Geeignete Container und Ausführungsformen eines Behältnisses sind z.B. in EP 2 123 289 A (supra) beschrieben. Die Inkubation erfolgt vorzugsweise bei 36 - 38 Grad Celsius, besonders bevorzugt unter Schwenken bis zu maximal 1 - 5 Stunden, besonders bevorzugt 3 Stunden. Nach 3 Stunden ist eine maximale gar 4,5-fache Anreicherung des Gehaltes an Interleukin-1-Rezeptor-Antagonisten erreicht. Nach 3 Stunden nimmt der Gehalt an inflammatorischen Interleukinen zu. Dieses dargelegte Verfahren erlaubt die vorteilhafte Herstellung eines autolog konditionierten Serums.

Daher betrifft die Erfindung ebenfalls einen Verfahrensschritt b.), wobei die Inkubation nach Schritt b.) bei 36 - 38 Grad Celsius unter Schwenken bis zu maximal 1 - 5 Stunden, besonders bevorzugt 3 Stunden erfolgt.

Insbesondere in einer bevorzugten Ausführungsform betrifft die Erfindung ein Arzneimittel oder Kosmetikum zur Haut- und Muskelbehandlung. Überraschender Weise zeigen Versuche, dass ein erfindungsgemäßes Serum zur Haut- und Muskelbehandlung einen palliativen oder curativen Effekt aufweist, insbesondere bei einer topischen bzw. lokalen Applikation an der Haut oder am Muskel bei Tier und Mensch.

Insbesondere zeigt sich eine Haut- und Muskelglättung, welches ebenfalls vorteilhaft zur Wundbehandlung verabreicht werden kann. Insbesondere wird vorteilhaft eine Narbenbildung verhindert. Weiterhin wird das Haarwachstum gefördert und Haarausfall (Alopecia) vermieden. Zudem erweist sich das erfindungsgemäße Serum geeignet für die Prophylaxe und Behandlung von Akne und Schuppenflechte (Psoriasis).

In einer weiteren bevorzugten Ausführungsform betrifft die Erfindung daher ein Arzneimittel, enthaltend ein erfindungsgemäßes Serum, zur Verwendung oder Anwendung, insbesondere in einem Verfahren oder Therapie, in der Behandlung von Wunden und Wundinfektionen, Alopecia, Akne, Psoriasis, wobei ein entsprechendes Arzneimittel im betroffenen Wund- oder Hautbereich verabreicht wird.

Weiterhin betrifft die Erfindung ein Arzneimittel, enthaltend ein erfindungsgemäßes Serum, zur Verwendung oder Anwendung, insbesondere in einem Verfahren oder Therapie, in der Prophylaxe und Behandlung von Muskel-, Sehnen- und Bänderverletzungen, insbesondere Muskel(faser)-, Sehnen- und Bänderrisse, wobei ein entsprechendes Arzneimittel verabreicht wird.

Die vorstehenden Verabreichungen erfolgen vorzugsweise im Rahmen einer lokalen Applikation, insbesondere einer topischen Applikation, wobei das erfindungsgemäße Serum subkutan verabreicht wird. Im Fall einer Wunde kann die subkutane Applikation im Wundbereich, insbesondere im nahen Wundbereich erfolgen.

In einer weiteren bevorzugten Ausführungsform betrifft die Erfindung ein Arzneimittel, enthaltend ein erfindungsgemäßes Serum, zur Verwendung oder Anwendung, insbesondere in einem Verfahren oder Therapie, Behandlung von Erkrankungen im Periodontium (Mundraum, insbesondere Zahnhalteapparat), wobei ein entsprechendes Arzneimittel verabreicht wird.

Im Rahmen dieser Erfindung sind solche Erkrankungen im Periodontium bevorzugt wie Zahnfleischentzündungen, Gingivitis, Stomatitis, Parodontitis apicalis.

Weiterhin ist bevorzugt, dass die Verabreichung des erfindungsgemäßen Serums in Kombination mit einer Laserbehandlung oder Lasertherapie erfolgt. Daher betrifft die Erfindung ein Arzneimittel oder Kosmetikum, wie vorstehend beschrieben, wobei eine lokale Laserbehandlung zur Unterstützung erfolgt. In einer bevorzugten Ausführungsform erfolgt die Laserbehandlung mittels sogenannter Lasernadeln oder Laserköpfen. Solche Lasernadeln können gesetzt werden, wie im Rahmen z.B. einer Akupunktur üblich (sogenannte "Laserakupunktur", Punktlaser, Laserpen), wobei die Laserbehandlung lokal in örtlicher Nähe zur lokalen Applikation des vorstehend genannten Arzneimittels oder Kosmetikums erfolgt. Solche Lasernadeln und Laserköpfe sind dem Fachmann hinreichend bekannt und können käuflich erworben werden. Ferner sind Laserquellen einschlägig beschrieben und im medizinischen Fachhandel erhältlich (z.B. MKW Therapie-Systeme GmbH, Sinzheim, Deutschland).

Die Wellenlängen des Lasers können variieren und ebenfalls können verschiedene Wellenlängen eingesetzt werden (sogenannter Laser-Shower). Entscheidend ist im Rahmen dieser Kombinationstherapie, dass eine zusätzliche Aktivierung des Immunsystems mittels der Lasertherapie erfolgt. Überraschenderweise hat sich gezeigt, dass der Behandlungserfolg des erfindungsgemäßen Arzneimittels oder Kosmetikums in Gegenwart einer solchen unterstützenden Lasertherapie sich verbessert.

Im Rahmen einer kosmetischen Anwendung wird die Faltung der Haut vermindert, welches besonders vorteilhaft im Rahmen einer Anti-Aging-Behandlung verwendet werden kann.

Daher betrifft die Erfindung ein Kosmetikum, insbesondere Anti-Aging-Mittel enthaltend ein erfindungsgemäßes Serum, welches vorzugsweise topisch oder lokal appliziert wird, insbesondere auf die Haut aufgetragen oder unter die Haut eingebracht wird, besonders bevorzugt mittels subkutaner Applikation.

Weiterhin betrifft die Erfindung eine Kombinationsbehandlung oder Kombinationstherapie und entsprechendes Verfahren oder Verwendung bzw. Anwendung, wobei zum verabreichten erfindungsgemäßen Arzneimittel oder Kosmetikum eine Laserbehandlung oder Lasertherapie erfolgt. Die Laserbehandlung kann vor, während oder nach der Verabreichung des Arzneimittels oder Kosmetikums erfolgen.

Daher betrifft die Erfindung ebenfalls ein Arzneimittel, enthaltend ein erfindungsgemäßes Serum, zur Verwendung oder Anwendung, insbesondere in einem Verfahren oder Therapie, in der Prophylaxe und Behandlung von entzündlichen Gelenks-, Muskel- und Sehnenerkrankungen, insbesondere Arthrose, Osteoarthritis, Arthritis, rheumatoide Arthritis, Polyarthritis, Morbus Bechterew, Muskelverhärtung, wobei eine lokale Applikation samt Laserbehandlung zur Unterstützung erfolgt.

Die subkutane Applikation erfolgt vorzugsweise mit einer Spritze.

Die Verabreichung kann einmal oder mehrmals wöchentlich erfolgen, vorzugsweise besteht zwischen zwei Applikationen eine 3 bis 4-tägige Pause. Die Dosis kann 0,1 bis 10 ml sein. Sofern erforderlich kann das erfindungsgemäße Serum mittels physiologisch unbedenklichen Zusatz- und Hilfsstoffen versetzt werden, wie z.B. eine physiologische Kochsalzlösung oder einem Bicarbonatpuffer. Das erfindungsmäßige Kosmetikum kann mittels einer üblichen Basiscreme verabreicht werden.

Die angegebenen Indikationen können z.B. Pschyrembel, de Gruyter Verlag, Berlin aktuelle Ausgabe entnommen werden.

## Patentansprüche

1. Arzneimittel zur Verwendung in der Prophylaxe und Behandlung von Muskel-, Sehnen-, Bänderverletzungen, insbesondere Muskel-, Sehnen-, Bänderriss, wobei ein Serum enthaltend Interleukin-1-Rezeptor-Antagonisten durch folgende Schritte hergestellt wird
a.) Blutentnahme,
b.) Inkubation des entnommenen Blutes in Gegenwart einer Oberfläche,
c.) Zentrifugation,
d.) Abtrennen des Überstandes unter Erhalt eines Serums, und lokale Applikation des Serums.

2. Arzneimittel zur Verwendung in der Prophylaxe und Behandlung von Erkrankungen im Periodontium, insbesondere Gingiva, Zahnzement, Alveolus dentalis, Wurzelhaut und Zahnfleisch, wobei ein Serum enthaltend Interleukin-1-Rezeptor-Antagonisten durch folgende Schritte hergestellt wird
a.) Blutentnahme,
b.) Inkubation des entnommenen Blutes in Gegenwart einer Oberfläche,
c.) Zentrifugation,
d.) Abtrennen des Überstandes unter Erhalt eines Serums, und
lokale Applikation des Serums im Mundraum.

3. Arzneimittel nach Anspruch 2 zur Verwendung in der Prophylaxe und Behandlung von Zahnfleischentzündungen, Gingivitis, Stomatitis, Parodontitis apicalis, wobei eine lokale Applikation des Serums im Mundraum erfolgt.

4. Arzneimittel zur Verwendung in der Prophylaxe und Behandlung von Wunden und Wundinfektionen, Alopecia, Akne, Psoriasis, wobei ein Serum enthaltend Interleukin-1-Rezeptor-Antagonisten durch folgende Schritte hergestellt wird
a.) Blutentnahme,
b.) Inkubation des entnommenen Blutes in Gegenwart einer Oberfläche,
c.) Zentrifugation,
d.) Abtrennen des Überstandes unter Erhalt eines Serums
lokale Applikation des Serums im Wundbereich oder Hautbereich.

5. Kosmetikum, wobei ein Serum enthaltend Interleukin-1-Rezeptor-Antagonisten durch folgende Schritte hergestellt wird
a.) Blutentnahme,
b.) Inkubation des entnommenen Blutes in Gegenwart einer Oberfläche,
c.) Zentrifugation,
d.) Abtrennen des Überstandes unter Erhalt eines Serums, und
lokale Applikation des Serums unter oder auf die Haut.

6. Anti-Aging Mittel enthaltend ein Kosmetikum nach Anspruch 5.

7. Mittel oder Kosmetikum nach einem der vorstehenden Ansprüchen, wobei die Inkubation nach Schritt b.) bei 36 - 38 Grad Celsius unter Schwenken bis zu maximal 1 - 5 Stunden, besonders bevorzugt 3 Stunden erfolgt und/oder die Oberfläche eine unebene oder raue Oberfläche ist, insbesondere ein Container ist, ggfs. enthaltend Kugeln oder Perlen.

8. Mittel nach einem der vorstehenden Ansprüchen, **dadurch gekennzeichnet, dass** eine lokale Laserbehandlung zur Unterstützung erfolgt.

9. Arzneimittel zur Verwendung in der Prophylaxe und Behandlung von entzündlichen Gelenks-, Muskel- und Sehnenerkrankungen, wobei ein Serum enthaltend Interleukin-1-Rezeptor-Antagonisten durch folgenden Schritte hergestellt wird
a.) Blutentnahme,
b.) Inkubation des entnommenen Blutes in Gegenwart einer Oberfläche,
c.) Zentrifugation,
d.) Abtrennen des Überstandes unter Erhalt eines Serums, und lokale Applikation des Serums, wobei eine Laserbehandlung zur Unterstützung erfolgt.

10. Arzneimittel nach Anspruch 9 zur Verwendung in der Prophylaxe und Behandlung von Arthrose, Osteoarthritis, Arthritis, rheumatoide Arthritis, Polyarthritis, Morbus Bechterew, Muskelverhärtung, wobei eine lokale Applikation samt Laserbehandlung zur Unterstützung erfolgt.

11. Mittel nach einem der vorstehenden Ansprüchen, **dadurch gekennzeichnet, dass** eine lokale Laserbehandlung in einer Vor- und / oder Nachbehandlung erfolgt.

12. Mittel nach einem der vorstehenden Ansprüchen, **dadurch gekennzeichnet, dass** die Laserbehandlung mittels einer Lasernadel erfolgt.

13. Mittel nach einem der vorstehenden Ansprüche, **dadurch gekennzeichnet, dass** die lokale Applikation eine topische Applikation, insbesondere eine subkutane Applikation ist.
